# EUROPEAN PATENT SPECIFICATION

(11) **EP 3 177 329 B1**
(45) Date of publication and mention of the grant of the patent: **18.12.2019**
(21) Application number: 15745201.2
(22) Date of filing: 03.08.2015
(51) Int. Cl.: A61L 15/44

(54) **SYSTEM FOR THE IMMEDIATE RELEASE OF ACTIVE AGENTS**
SYSTEM ZUR UNMITTELBAREN FREISETZUNG VON WIRKSTOFFEN
SYSTÈME POUR LA LIBÉRATION IMMÉDIATE DE PRINCIPES ACTIFS

(30) Priority: 04.08.2014 ES 201431189
(43) Date of publication of application: 14.06.2017
(73) Proprietor: Hospital Sant Joan de Deu, 08950 Esplugues de Llobregat Barcelona (ES); Universitat Politècnica De Catalunya, 08034 Barcelona (ES)
(72) Inventor: TORNERO GARCIA, Jose Antonio, 08232 Viladecavalls (ES); MONTERO CARCABOSO, Angel, 08174 Sant Cugat del Vallés (ES); CANO CASAS, Francesc, 08223 Terrassa (ES); BERTRAN I LLAVINA, Joan, 08350 Arenys De Mar (ES)
(74) Representative: Carvajal y Urquijo, Isabel
(86) International application number: PCT/EP2015/067763
(87) International publication number: WO 2016/020306

(56) References cited:
- EP-A1- 2 644 191
- WO-A1-02/00149
- JP-A- 2008 179 629
- US-A1- 2002 096 246
- DATABASE WPI Week 200934 Thomson Scientific, London, GB; AN 2009-G92551 XP002744999, & CN 101 390 814 A (UNIV SOUTHEAST) 25 March 2009 (2009-03-25)

## Description

The present invention relates to a system of immediate release of active agents. In particular, it relates to a nonwoven fabric membrane for topical use on the skin, which comprises polymeric nanofibres and a high amount of, at least, one active agent, and to a process for the preparation of said membrane. It also relates to cosmetic or sanitary products and kits comprising said membranes and to their therapeutic and cosmetic uses.

### BACKGROUND OF THE ART

The skin is the biggest organ of the human and animal body that consists of two different layers, the epidermis and the dermis. The epidermis is the outer layer and mainly consists of keratinocytes and of a significant amount of melanocytes, whose main function is the production of melanin that plays an important role for cosmetic and sun protection purposes. Furthermore, the dermis, or the inner layer, consists of a network of collagen and elastic fibres; it is thicker than the epidermis and provides the skin with its elasticity and consistency.

Skin has multiple functions. These include the barrier function, which prevents the entry of external substances or organisms and the loss of internal components, and also acts as a filter for ultraviolet radiation. Other functions of the skin are the immune function, to prevent fungal, bacterial, viral infections, autoimmune and neoplastic diseases; the restorative function in wound healing, skin ulcers and cell damage caused by ultraviolet radiation; the vascular function that allows the absorption of nutrients, the removal of metabolic wastes and intervenes in the regulation of body temperature; the sensory or communicative function with the outside environment and the function of relationship or care to other individuals.

The healing of skin wounds is a complex process in which the skin repairs itself after injury. In normal skin, the epidermis and the dermis exist in stationary state equilibrium, forming a protective barrier against the external environment. Once the barrier is broken, the physiological process of wound healing immediately starts.

Furthermore, despite the important protective function exerted by the melanin to avoid the harmful effects of solar radiation on our skin, the irregular accumulation of this pigment and the formation of abnormally pigmented areas can cause not only an aesthetic problem but also a skin health problem. It has been proved that apart from the ethnic differences, the sun's ultraviolet radiation (UVR) is the main cause of variations in skin pigmentation. Many hyperpigmentation disorders, characterised by an abnormal darkening of the skin or the appearance of hyperpigmented located areas, are caused either by an increase in the synthetic melanin levels or by an increase of melanocytes in the epidermis.. This increase in melanin production is the cause of very common disorders such as for example sunspots or maculas, also Addison's disease or post-inflammatory hyperpigmentation.

Both in the therapeutic field, for example in wound healing, and in the cosmetic field, for example to improve the appearance of the skin, topical application of active agents on the skin is very important. Topical administration has the advantage of allowing the reduction of systemic side effects compared with parenteral or oral administration of the active agents. In topical administration, the first hepatic step and plasma level fluctuations caused by repeated oral administration of active agents that are rapidly eliminated, are avoided.

Traditionally, methods for topical application of active agents on the skin are based on the dissolution of the active agent in a suitable solvent and the application of the solution to the skin or in the application of emulsions containing the active agent, thereby increasing absorption through the skin. However, these methods have various difficulties, such as the choice of solvents that are compatible with the active agents that do not cause irritation and have a nice tactile sensation. Furthermore, the emulsions are physically and chemically unstable and they are easy to break by organic or inorganic pollutants and they are often sensitive to light, therefore their storage for long periods of time is expensive and difficult. Moreover, in many cases a great amount of emulsifiers is required to contain a sufficient amount of active agent, which can cause skin irritation. And finally, in the case of active agents that are unstable in solution, such as vitamin C, these methods are not suitable since continuous contact of the active agent with water causes oxidation and degradation thereof.

Polymeric nanofibres are topical carriers for the administration of active agents and offer advantages such as low toxicity and high biocompatibility. Document JP2008179629 describes a network of polymeric nanofibres containing cosmetic active agents, such as vitamin C, which are slowly and gradually released from the membrane by addition of a solvent. Polymeric nanofibres described herein are obtained by an electrospinning process from a mixture containing the cosmetic active agent and the polymer solution that forms the nanofibres. This process has several disadvantages. Firstly, the solvent employed must be suitable for both the polymer that will form the nanofibres and the active agent. Since it is not possible to find an optimal solvent for both products, the nanofibres lose regularity, this fact being seen with the naked eye. In addition, since the solution is not totally homogeneous, regular dosage of active ingredient in the membrane cannot be ensured. On the other hand, the particles of active agent are coated by the polymer of the nanofibres, so that said release system does not allow an immediate release of the active agent, as in order to release the active agent contained in the nanofibres, said nanofibres need to be, at least, partially degraded. Inventors have found that the nanofibre sheet described herein is only capable of releasing about 12 % by weight of the active agent contained in it 120 minutes after its application.

CN101390814 discloses a cosmetic mask made of a nonwoven fabric comprising electrospun nanofibers made of a polymer selected in a list comprising e.g. cellulose, hyaluronic acid, collagen and of at least one active agent e.g. vitamin C arranged externally to the polymeric nanofibers. In example 2 the amount of active is 50 wt%. The active ingredient(s) is entrapped inside the electrospun nanofibers. The only process described in said document is a process which comprises electrospinning a polymer mixture with a bioactive agent.

The immediate release of the active agent enables rapid absorption and, consequently, rapid appearance of cosmetic or therapeutic effects of the active agents. Furthermore, this form of release may be particularly suitable for acute conditions such as in wound healing.

Therefore, there is a need to develop systems of immediate release of topical active agents on the skin with a rapid effect that overcome the problems of the state of the art, in particular, problems of low absorption or poor release of the active agent, skin irritation and/or stability of the active agent.

### SUMMARY OF THE INVENTION

Inventors have developed a new stable system of topical release of therapeutic or cosmetic active agents on the skin containing a high amount of at least one active agent that remains attached to the membrane until its application, allowing its storage, preferably under an inert atmosphere and in the absence of water. The fact that the membrane contains a high amount of active agent, equal to or greater than 25 % by weight with respect to the total weight of the membrane, allows the increase of the therapeutic or cosmetic activity on the skin.

In addition, the system for the release of active agents of the invention substantially allows immediate release of the total amount of active agent contained in the membrane after contacting said membrane with a suitable biocompatible solvent system in which the active agent is completely solubilised. Therefore, the nonwoven fabric membrane of the present invention has the advantage of providing a fast effect, which can be implemented depending on the needs and which remains chemically and physically stable while its application is not required.

On the other hand, the release system of the invention allows to control the dosage of active agent per surface area of the skin which is an advantage over other formulations such as topical emulsions which may lead to an incorrect dosage of the active agent, either if insufficient or too high. Also, as it is not an occlusive system and it only contains biocompatible components, it does not cause any adverse effect on the skin. Furthermore, the release system of the invention has a pleasant feel on the skin and leaves no undesirable residues when being removed after its application.

Additionally, the system of the invention has the advantage that when the active agent is unstable in solution, it enables the active agent to remain stable in the membrane being the stability of the active agent in the membrane comparable to the stability of the dry active agent in solid state. Furthermore, the final membrane can be stored in a protective atmosphere for the active agent (for example, free of oxygen and/or moisture).

Thus, a first aspect of the invention relates to a nonwoven fabric membrane for topical use on the skin, according to claim 1.

In the context of the invention, the term "nonwoven fabric membrane" relates to a porous sheet (film) of arbitrarily-deposited polymeric nanofibres on a solid surface (collector) and optionally entangled by one or more of following methods: physical methods such as adhesion with a biocompatible glue, melting or solvent excess and mechanical methods such as needle punching and the interlacing by water or pneumatic injectors. The membrane fibres may or may not be interconnected, they may be cut or continuous fibres and may comprise only one material or different materials, either as a combination of different fibres or as a combination of similar fibres of different materials. These different configurations can be arranged in the same membrane in different layers during the production process.

In a particular embodiment, optionally in combination with one or more features of the various embodiments described in this invention, the membrane of the invention has a thickness from 0.1mm to 3mm, more particularly from 0.3mm to 1mm.

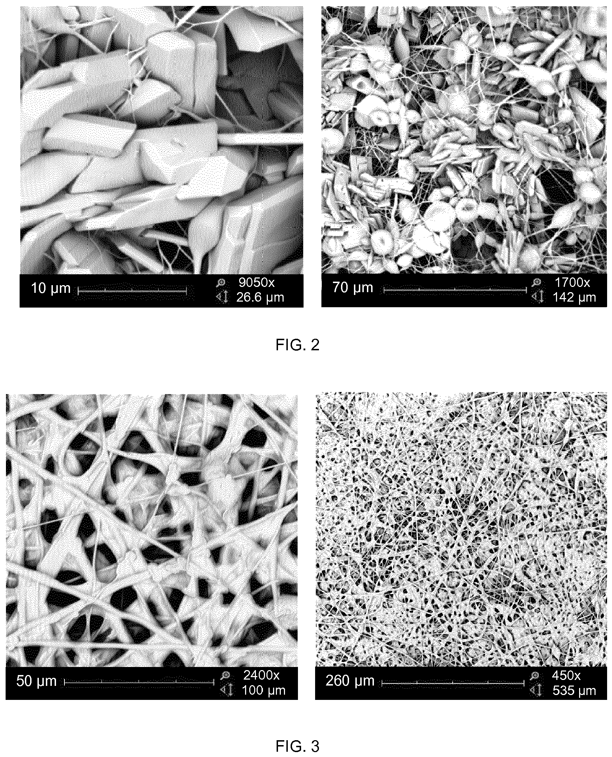

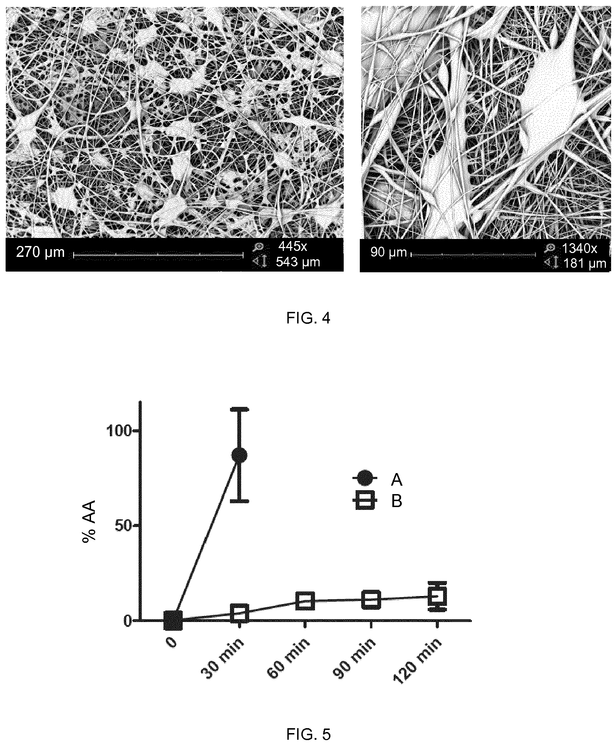

The total amount of active agent contained in the nonwoven fabric membrane is externally arranged to the polymeric nanofibres of the membrane, as illustrated in FIG. 2 and FIG. 3, unlike the membranes obtained by the electrospinning of a mixture of both, the polymer and active agent, where the active agent is internally arranged in the polymeric nanofibres (illustrated in FIG. 4). Thus, the nonwoven fabric membrane of the invention releases the active agent in the presence of a suitable solvent system, i.e., the mechanism of release occurs through the solubilisation of the active agent and not by degradation of the polymeric nanofibres. In addition, the active agent that externally coats the polymeric nanofibres provides the membrane with consistency and cohesion, thus improving the handling resistance of the membrane (see FIG. 2 and FIG. 3).

In another particular embodiment, optionally in combination with one or more features of the various embodiments described in this invention, the polymeric nanofibres of the membrane are electrospun nanofibres, i.e. nanofibres obtained by an electrospinning process.

In another particular embodiment, optionally in combination with one or more features of the various embodiments described in this invention, the polymeric nanofibres of the membrane have an average diameter from 50 to 2000nm, more particularly from 200 to 1200 nm, from 400 to 800 nm and an average diameter of 500 nm. For the purposes of the present invention, the term "average diameter" relates to the diameter obtained by the arithmetic average of different measurements, particularly 50 measurements, on an image obtained by electronic scanning microscope.

The polymeric nanofibres of the nonwoven fabric membrane of the invention consist of one or more biocompatible polymers. Examples of suitable polymers for forming the nanofibers may be a polyester, a polyanhydride a polyphosphazene, a polyether, etc. In a particular embodiment, optionally in combination with one or more features of the various embodiments described in this invention, the nanofibres of the invention comprise one or more polymers selected from the group consisting of polyglycolic acid (PGA), poly-D,L-lactic acid (PLA), poly-D, L-lactide-co-glycolide (PLGA), polycaprolactone (PCL), polydioxanone, polyvinylalcohol, collagen, cellulose, hyaluronic acid, polyamide, polyester, polyurethane, polypropylene, elastanes among other electrospun polymers and a combination thereof. More particularly, the nanofibres of the invention comprise poly-D, L-lactic acid (PLA).

As indicated above, the membrane of the invention comprises, besides the polymeric nanofibres, at least one active agent in an amount from 25 to 80 % by weight with respect to the total weight of the nonwoven fabric membrane. In a particular embodiment, optionally in combination with one or more features of the various embodiments described in this invention, the active agent is comprised in the membrane in an amount from 30 to 70 %, more particularly from 30 % to 55 % with respect to the total weight of the nonwoven fabric membrane.

The active agent of the membrane may be in solid form, arranged among the fibres, being able to form, in this case, crystals (with a crystalline or amorphous structure), or in liquid form, embedded in the surface of the fibres. In a particular embodiment, optionally in combination with one or more features of the various embodiments described in this invention, the nonwoven fabric membrane of the invention comprises a therapeutic active agent which can be used both in the pharmaceutical field and in the veterinary field. In another particular embodiment, optionally in combination with one or more features of the various embodiments described in this invention, the nonwoven fabric membrane of the invention comprises a cosmetic agent. In another particular embodiment, optionally in combination with one or more features of the various embodiments described in the present invention, the nonwoven fabric membrane of the invention comprises more than one active agent, more particularly the membrane comprises a second active agent which is soluble in a suitable biocompatible solvent system, wherein the membrane is capable of immediately releasing said second active agent when contacted with an appropriate amount of suitable biocompatible solvent system wherein the second active agent is completely solubilised.

For purposes of the present invention, the term "therapeutic" means that it is therapeutically useful, for example, in the treatment, remission or attenuation of a disease state, physiological state, or their symptoms or for the evaluation or diagnosis thereof. The term "cosmetic agent" relates to a use intended to provide, primarily, an aesthetic and/or comfort effect, in particular, to improve the appearance of skin, and specifically the properties of the skin.

Examples of active agents include, without limitation, the L-Ascorbic acid (vitamin C), resveratrol, quercetin, extract of *Boswellia serrata,* curcumin, lactoferrin, hydroquinone, kojic acid, collagen, cinnamic acid, benzoyl peroxide, tropolone, catechol, mercaptamine, niacinamide, tocopherol, ferulic acid, azelaic acid, botulin toxin, urea, as well as xanthine, retinoids, α-hydroxy acids, β-hydroxy acids, antibiotics, anti-inflammatory agents, healing agents, α2-adrenergic inhibitors, β-adrenergic agonists, aromatase inhibitors, antiestrogens, corticosteroids, mucopolysaccharides, oestrogens, isoflavones or derivatives or salts thereof.

In a particular embodiment, optionally in combination with one or more features of the various embodiments described in this invention, the nonwoven fabric membrane of the invention comprises the L-ascorbic acid (Vitamin C) or resveratrol as active agent.

As mentioned above, the nonwoven fabric membrane of the invention is capable of immediately releasing the active agent thereof so that it is absorbed on the skin when the membrane is contacted with an appropriate amount of a suitable biocompatible solvent system wherein the active agent is completely solubilised. The active agent must be soluble in said solvent system and the solvent system must be provided in an appropriate amount sufficient to completely dissolve the total content of the active agent of the membrane. Said amount will depend on the type of active agent and the type of solvent system used. If the membrane comprises more than one active agent, each active agent must be soluble in a suitable biocompatible solvent system and must be able to be solubilised in the same when the nonwoven fabric membrane is contacted with an appropriate amount thereof. In this case, the suitable solvent system can be the same or different for each active agent.

The solvent system that solubilises the active agent may comprise one or more solvents selected from the group consisting of water, hydrophilic solvents, lipophilic solvents and mixtures thereof. The term "hydrophilic" solvent relates to a solvent having affinity with water and being capable of creating hydrogen bonds, so that it is miscible in water and other polar solvents. The term "lipophilic" solvent relates to a nonpolar solvent that has no affinity with water and has little or no ability to form hydrogen bonds. Thus, a lipophilic solvent is miscible in fats, oils, lipids, and other nonpolar solvents.

Examples of suitable solvents for the release of the membrane active agents include, without limitation, water; (C₂-C₁₂)alcohols, (C₂-C₁₂)glycols, (C₂-C₆)-glycol (C₂C₆)ethers; triglycerides of capric/caprylic acid, vegetable oils, mineral oils, animal fat, etc., fractions and mixtures thereof.

The term (C₂-C₁₂) alcohol relates to a hydrocarbon compound containing from 2 to 12 carbon atoms and one or more hydroxyl groups (OH). The term (C₂-C₁₂) glycol relates to an alcohol containing from 2 to 12 carbon atoms and two hydroxyl (OH) groups which are bonded to adjacent carbon atoms. The term (C₄-C₁₂) glycol ether relates to a hydrocarbon compound containing from 4 to 12 carbon atoms and at least two hydroxyl (OH) and at least one ether group (O), wherein each of the hydroxyl groups (OH) and the ether group (O) are independently bounded to adjacent carbon atoms. Nonlimiting examples of alcohols, glycols and glycol ethers include for example, ethanol, ethylene glycol, propylene glycol, butylene glycol, diethylene glycol, tetraethylene glycol, 1,2,3-propanetriol (glycerin) or benzyl alcohol.

The term "biocompatible solvent system" herein relates to a solvent system which, in contact with the skin, does not cause a toxic or harmful immune response or an undue immune response.

For the nonwoven fabric membrane to release the active agent comprised therein, the active agent should fully be solubilised in a biocompatible solvent system. In a particular embodiment, optionally in combination with one or more features of the various embodiments described in this invention, the solubilisation takes place at a temperature between 25 to 36 °C.

The term "completely solubilised" relates herein to the active agent as part of the membrane, when contacting a solvent system in which it is soluble, that forms a solution with the solvent system.

The solubility of each active agent is different for each solvent. Also, for a concrete solvent system the solubility of each active agent is different. The expert in the art will be able to easily and routinely determine the suitable biocompatible solvent system for each active agent to be released and the appropriate amount thereof so that the active agent is completely solubilised therein.

Alternatively, the release of the active agent can occur after contacting the nonwoven fabric membrane that has been previously defined, with a topical composition comprising a suitable biocompatible solvent system wherein the active agent is completely solubilised together with one or more excipients or suitable topical carriers. In general, any topical composition may be suitable for the purposes of the invention provided it has a suitable viscosity and it penetrates the membrane in a relatively short period time, such as a 30-minute period, more particularly a 15-minute period.

Examples of suitable topical composition to release the active agent of the membrane include liquid, solid and semi-solid compositions, such as aqueous solutions or suspensions, anhydrous compositions, oil-in-water emulsions, water-in-oil emulsions, either being macroemulsions, microemulsions or nanoemulsions, oily droplets in solutions, micelles, liposomes, ethosomes, and the like. Said compositions can be in the form of a liquid, a gel, a paste, a cream, an emulsion, a lotion, a foam, a spray, a patch, a stick, or any other known form in the art. In addition, said compositions can be prepared according to methods that are well known in the state of the art. Suitable excipients and/or carriers, and their amounts can be easily determined by one expert in the art according to the type of formulation being prepared.

Optionally, the topical compositions comprising the suitable biocompatible solvent system to release the active agent of the membrane may also contain one or more active agents such as those previously defined for the membrane.

Thus, such topical compositions may comprise, apart from a suitable solvent system, skin toning agents or other agents that are beneficial to the skin.

The term "topical excipients or carriers" relates to acceptable materials for skin use. Each component must be acceptable in the sense of being compatible with the other ingredients of the topical composition. It must also be suitable for use in contact with human or animal skin without undue toxicity, incompatibility, instability, allergic response, among others.

The term "immediate" release relates to the nonwoven fabric membrane of the invention substantially releasing the full dose of the active agent contained therein in a short period of time after contacting the membrane with a suitable solvent system wherein the active agent is completely solubilised, in particular at a temperature between 25 - 36 °C. The nonwoven fabric membrane of the invention is able to release an amount equal to or greater than 70 %, equal to or greater than 80 %, equal to or greater than 90 % of the total weight of the active agent comprised therein in a of 30-minute period after contacting the membrane with the suitable solvent system. In a particular embodiment, the nonwoven fabric membrane of the invention is able to release an amount equal to or greater than 50 %, equal to or greater than 60 %, equal to or greater than 70 %, equal to or greater than 80 %, equal to or greater than 90 % of the total weight of the active agent comprised therein in a of 10-minute period after contacting the membrane with the suitable solvent system.

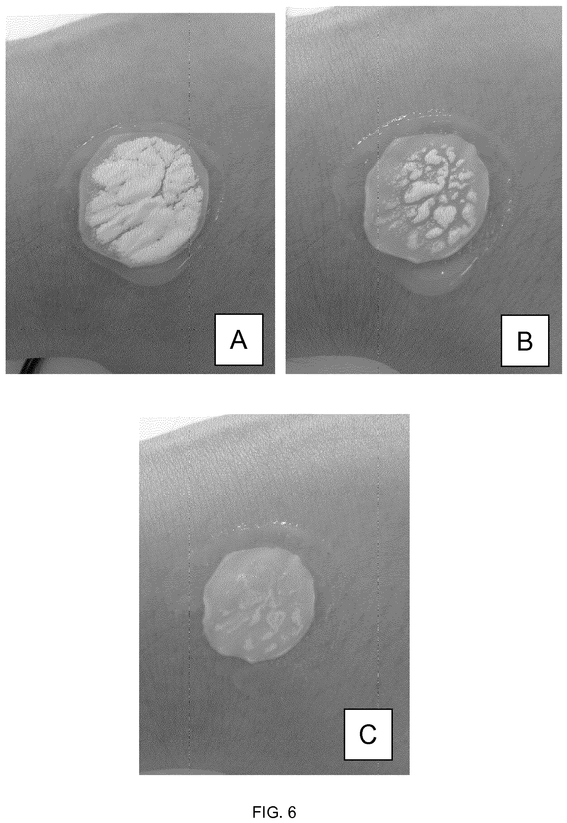

FIG. 6 shows a particular embodiment of the application of a nonwoven fabric membrane of the invention on human skin, said membrane having 15 mm diameter, loaded with 10 mg of vitamin C on a gel of carbopol at different stages of the membrane wetting: 5 minutes (A), 15 minutes (B) and 25 minutes (C) after contacting the membrane with the gel.

In a particular embodiment, optionally in combination with one or more features of the various embodiments described in this invention, the invention relates to a nonwoven fabric membrane comprising polymeric nanofibres consisting of one or more polymers selected from the group of PLA, polyester, polypropylene and polyethylene, particularly nanofibres constituted of PLA; and L-asorbic acid in an amount from 25 to 80 %, more particularly from 30 to 70 %, more particularly from 40 to 60 % by weight with respect to the total weight of the nonwoven fabric membrane; wherein the suitable biocompatible solvent system is water, more particularly water as part of a topical composition in the form of an hydrophilic gel, for example an aqueous gel of carbopol at 0.5-2 % weight/volume carbopol, or as part of a topical composition such as oil in water (O/W) such as a cream, an emulsion or a lotion; and the appropriate amount of biocompatible solvent system is from 0.05 to 0.5 mL/cm².

In a particular embodiment, optionally in combination with one or more features of the various embodiments described herein, the invention relates to a nonwoven fabric membrane comprising polymeric nanofibres consisting of one or more polymers selected from the group of PLA , polyester, polypropylene and polyethylene, particularly consisting of PLA nanofibres; and resveratrol in an amount from 25 to 80 %, more particularly from 25 to 60 %, more particularly from 25 to 50 % by weight with respect to the total weight of the nonwoven fabric membrane; where the suitable biocompatible solvent system is selected from water, (C₂-C₁₂)alcohol (C₂-C₁₂)glycol and mixtures thereof, more particularly the biocompatible solvent system is a mixture of water, (C₂-C₁₂)alcohol, (C₂-C₁₂)glycol being part of a hydroalcoholic gel, for example a hydroalcoholic gel of carbopol containing water, 1-2 % weight/volume carbopol, 10-25 % weight/volume of ethanol or propylene glycol; and the appropriate amount of the biocompatible solvent system is from 0.05 to 0.5 mL/cm².

A cosmetic or sanitary product comprising the nonwoven fabric membrane such as previously defined, is also part of the invention. In a particular embodiment, optionally in combination with one or more features of the various embodiments described in this invention, the cosmetic or sanitary product comprises a support on which the nonwoven fabric membrane of the invention may or may not be bound. Examples of cosmetic or sanitary products include without limitation, gauzes, sticking plasters, dressings, bandages, sponges, wipes, masks and the like.

A kit comprising the nonwoven fabric membrane previously defined and a suitable biocompatible solvent system capable of solubilising the active agent when the membrane is contacted therewith, is also part of the invention. Optionally, said solvent system may be part of a topical composition.

Particular embodiments relating to the biocompatible solvent system listed above are also particular embodiments of the kit of the invention.

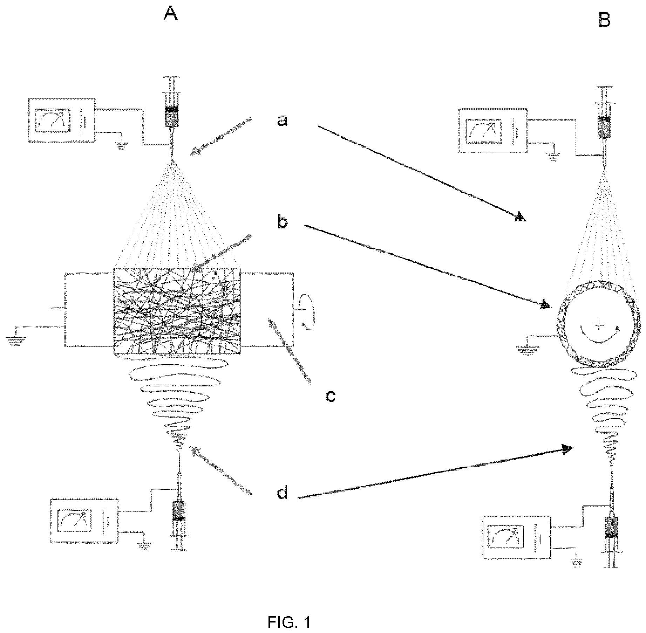

The nonwoven fabric membrane of the invention is prepared by an electrospinning/elctrospray process. The device used consists of an electrospinning system and an electrospraying system that run simultaneously. The electrospinning generates polymeric nanofibres from a solution of one or more polymers that are deposited on a collector (which may be a rotating cylinder or any other collector that oscillates between the deposition zones of the electrospinning and the electrospraying). The spray system, such as electrospray, generates microdrops of a solution containing the active agent. Said drops are deposited on the nanofibres that coat the collector. Once the solvent of the drops evaporates, the active agent is in solid form, forming either a coating to the fibres or solid particles that can have crystalline or amorphous structure, depending on the ultimate disposition of the nature of the product, its viscosity and the ability to form crystals. In FIG. 1 a diagram (A: side view; B: front view) of a particular embodiment of a system for the preparation of the nonwoven fabric membranes of the invention is shown, wherein a) is the electrospray system of the active agent, b) the obtained nonwoven fabric membrane, c) the rotatory collector and d) is the electrospinning system of the polymeric nanofibres.

The spray system for depositing the active agent operates in the flow rates and viscosities determined by the active agent itself, and may be for example, an electrospray system, pneumatic spray, ultrasonic spray or combined spray pneumatic/ultrasound. In a particular embodiment, optionally in combination with one or more features of the various embodiments described in this invention, the spray system used is electrospray.

Thus, another aspect of the invention relates to a process for the preparation of the defined nonwoven fabric membrane as defined above, which comprises the following steps:
a) preparing a solution of one or more biocompatible polymers in a suitable solvent system;
b) preparing a solution of the active agent in a suitable solvent system, in which the polymer or polymers of step a) are insoluble;
c) performing the electrospinning of the solution of step a) and, simultaneously, spraying the solution of step b) with an appropriate flow rate of the solution of step b) and during an appropriate time to obtain a nonwoven fabric membrane comprising the active agent in an amount from 25 to 80 % by weight with respect to the total weight of the nonwoven fabric membrane; and
d) optionally drying the nonwoven fabric membrane obtained in step c). This drying step can be performed by evaporating any remaining solvent that has been employed.

Some examples of suitable solvents for the solution of one or more biocompatible polymers of step (a) include, without limitation, dichloromethane, chloroform, ethyl acetate, acetone, dimethylformamide, dioxane, dimethylsulfoxide (DMSO) or mixtures thereof. The solvent may include additives to improve the electrospinning process, as surfactant agents or salts.

In a particular embodiment, optionally in combination with one or more features of the various embodiments described in this invention, the polymer concentration in the polymeric solution of step a) is between 7 to 18 % weight/weight with respect to the total weight of the solution.

For the purposes of the invention, in step b), a solvent system capable of dissolving the active agent but that is not capable of solubilising the polymer or polymers employed in step a) is used. Thus, the solvent system of step b) is different from the solvent system of step a). Also, the solvent of step b) must be compatible with the desired active agent so as not to cause its degradation. Some examples of suitable solvents for the preparation of the solution of active agent include, without limitation, ethanol, water and the like or mixtures thereof.

Generally, the process of electrospinning/electrospray is performed during a necessary time, with a flow polymeric solution and a flow rate of active agent solution such as to obtain the nonwoven fabric membrane containing active agent comprised in an amount from 25 to 80 % by weight with respect to the total weight of the nonwoven fabric membrane.

Furthermore, the process can be carried out in different steps wherein successive layers of membrane with different concentrations of active agent are generated.

The expert in the art will be able to routinely adjust the suitable solvents both to prepare the polymeric solution such as the active agent solution, as well as the suitable parameters of the electrospinning process to obtain a nonwoven fabric membrane with appropriate characteristics for the desired application.

After the solvents have been evaporated from the membrane, said membrane can be directly applied to the skin. In a particular embodiment, the process of the invention comprises drying the nonwoven fabric membrane obtained after the electrospinning/electrospray process, more particularly the drying step is performed by leaving to dry the solvents employed in the processes of electrospinning and electrospraying.

Another aspect of the invention relates to a nonwoven fabric membrane for topical use on the skin, which comprises polymeric nanofibres and an active agent in an amount from 25 to 80 % by weight with respect to the total weight of the nonwoven fabric membrane, wherein the active agent is capable of immediately releasing itself when completely solubilised in a biocompatible solvent system, obtainable by a process which comprises the following steps:
a) preparing a solution of one or more biocompatible polymers in a suitable solvent system;
b) preparing a solution of the active agent in a suitable solvent system, in which the polymer or polymers of step a) are insoluble;
c) performing the electrospinning of the solution of step a) and, simultaneously, spraying the solution of step b) with an appropriate flow rate of the solution of step b) and during an appropriate time to obtain a nonwoven fabric membrane comprising the active agent in an amount from 25 to 80 % by weight with respect to the total weight of the nonwoven fabric membrane; and
d) optionally drying the nonwoven fabric membrane obtained in step c).

The nonwoven fabric membrane "obtainable by" the process of the invention defined above is used herein to define the membrane by its preparation process and relates to the membrane obtainable by the preparation process which comprises the steps a), b), c) and d) above defined. For the purposes of the invention, the expressions "obtainable", "obtained" and equivalent expressions are interchangeably used, and in any case the expression "obtainable" includes the expression "obtained".

The nonwoven fabric membrane of the invention is useful in different cutaneous treatments and in beauty treatment.

When the nonwoven fabric membrane of the invention comprises a therapeutic agent it is useful in disease treatments or skin disorders. Thus, another aspect of the invention relates to the nonwoven fabric membrane, as previously defined, wherein the active agent is a therapeutic agent, for use in the treatment of a disease, disorder or abnormal condition of the skin. This aspect of the invention can also be formulated as a use of an active agent for the preparation of a nonwoven fabric membrane for topical use on the skin, which comprises polymeric nanofibres and an active agent in an amount from 25 to 80 % by weight with respect to the total weight of the nonwoven fabric membrane, for the treatment of a disease, disorder or anomalous condition of the skin, wherein the active agent is a therapeutic agent that is soluble in a suitable biocompatible solvent system, and wherein the nonwoven fabric membrane is capable of releasing the active agent immediately when contacted with an appropriate amount of the suitable solvent biocompatible system in which the active agent is completely solubilised.

Another aspect of the invention relates to the product comprising the nonwoven fabric membrane previously defined, wherein the active agent is a therapeutic agent, for use in the treatment of a disease, disorder or abnormal condition of the skin. This aspect of the invention can also be formulated as a use of an active agent for the preparation of a sanitary or cosmetic product comprising a nonwoven fabric membrane for topical use on the skin comprising polymeric nanofibres and an active agent in an amount from 25 to 80 % by weight with respect to the total weight of the nonwoven fabric membrane, for the treatment of a disease, disorder or anomalous condition of the skin, wherein the active agent is a therapeutic active agent that is soluble in a suitable biocompatible solvent system, and wherein the nonwoven fabric membrane is capable of releasing the active agent immediately when contacted with an appropriate amount of the suitable solvent biocompatible system in which the active agent is completely solubilised.

The invention also relates to a method for the treatment of a mammal, including a human, suffering from a disease, disorder or abnormal condition of the skin, said method comprising the topical application on the skin of said mammal, including a human, of the nonwoven fabric membrane or the previously defined product, where the active agent in a therapeutic agent, .

Another aspect of the invention relates to a kit as defined above for use in the treatment of a disease, disorder or anomalous condition of the skin, wherein the active agent is a therapeutic agent.

In a particular embodiment, optionally in combination with one or more features of the various embodiments described herein, the disease, disorder or abnormal skin condition is selected from the group consisting of cellulitis, acne (including acne vulgaris and cystic acne), skin aging, hyperpigmentation (including discrete and mottled hyperpigmentation), keratoses (including actinic keratosis, solar keratosis, and seborrheic keratosis), dandruff, warts, photodamaged skin, chronic skin diseases (such as psoriasis, dermatitis, dermatitis including atopic and seborrheic dermatitis), dryness, ichthyosis, wound healing and skin infections caused by viruses, fungi or bacteria. For the purposes of the present description, the term "treatment" means compensation for a physiological dysfunction and more generally a reduction or elimination of an undesirable disorder, whose manifestation is especially a consequence of this dysfunction.

In addition, as it has been mentioned above, the nonwoven fabric membrane of the invention can also be used for cosmetic purposes, when the active agent is a cosmetic agent. Thus, the present invention also relates to a cosmetic use of the nonwoven fabric membrane that has been previously defined, or of the product comprising the membrane previously defined, wherein the active agent is a cosmetic agent, as a skin care agent.

It is also part of the invention, a cosmetic method for the skin care of a mammal, including a human, said method comprising the topical application on the skin of said mammal, including a human, of the nonwoven fabric membrane or the product defined above, wherein the active agent is a cosmetic agent.

In a particular embodiment, optionally in combination with one or more features of the various embodiments described herein, skin care comprises improving at least one of the following symptoms: aging, acne, wrinkles, skin blemishes, cellulitis, skin imperfections, roughness, scaling, dehydration, strain, cracking and lack of elasticity, among others.

Moreover, the nonwoven fabric membrane of the invention or the cosmetic or sanitary product as defined above may also be useful in whitening skin or tattoo removal.

Thus, the present invention also relates to a cosmetic use of the nonwoven fabric membrane or of the product previously defined, wherein the active agent is a cosmetic agent, as a skin whitening agent. The invention also relates to a cosmetic method for the skin whitening of a mammal, including a human, said method comprising the topical application on the skin of said mammal, including a human, of the nonwoven fabric membrane or the product defined above, wherein the active agent is a cosmetic agent.

The invention also relates to a cosmetic use of the nonwoven fabric membrane or of the product previously defined, wherein the active agent is a cosmetic agent, for tattoo removal. The invention also relates to a cosmetic method for removing tattoos from the skin of a mammal, including a human, said method comprising the topical application on the skin of said mammal, including a human, of the nonwoven fabric membrane or the product defined above, wherein the active agent is a cosmetic agent.

In a particular embodiment, optionally in combination with one or more features of the various embodiments described in this invention, the membrane is removed from the skin after its application on the skin.

Throughout the description and claims the word "comprise" and its variations are not intended to exclude other technical characteristics, additives, components or steps. Furthermore, the word "comprise" includes the case "consists of". For experts in the art, other objects, advantages and features of the invention will partly arise from the description and partly from the practice of the invention. The following examples and drawings are provided as an illustration, and are not intended to be limiting of the present invention. Moreover, the present invention covers all possible combinations of particular and preferred embodiments herein specified.

### BRIEF DESCRIPTION OF THE DRAWINGS

FIG. 1 shows a diagram (A: side view; B: front view) of a particular embodiment of a system for the preparation of the nonwoven fabric membranes of the invention, wherein a) is the electrospray system of the active agent, b) the obtained nonwoven fabric membrane, c) the rotatory collector and d) is the electrospinning system of the polymeric nanofibres.
FIG. 2 shows images obtained by scanning electron microscopy (SEM) of the membrane of the invention of Example 1 at different magnifications.
FIG. 3 shows images obtained by scanning electron microscopy (SEM) of the membrane of the invention of Example 2 at different magnifications.
FIG. 4 shows images obtained by scanning electron microscopy (SEM) of the membrane of the Comparative Example 1, prepared according to the process of the state of the art, at different magnifications.
FIG. 5 shows the amount of L-ascorbic acid released as fraction (%) with respect to the total theoretical amount of a nanofibre membrane prepared according to Example 1 (A) and of a nanofibre membrane prepared according to Comparative Example 1 (B) over time.
FIG. 6 shows a particular embodiment of the application of a nonwoven fabric membrane of the invention on human skin, said membrane having 15 mm diameter, loaded with 10 mg of vitamin C on a gel of carbopol at different stages of the membrane wetting: 5 minutes (A), 15 minutes (B) and 25 minutes (C) after contacting the membrane with the gel.

### EXAMPLES

### Technique of High-Performance Liquid Chromatography (HPLC) for quantification of L-ascorbic acid.

Selected conditions for the HPLC technique of detection of L-ascorbic acid were the following ones:
- HPLC Shimadzu equipment consisting of isocratic pump LC 20 AD, SIL HT 20A autoinjector, 20A SPD UV detector.
- HPLC Column Tracer Excel 120 ODSA, particle size 5 µm, 15 x 0.4 cm.
- Mobile phase 65 % methanol, 35 % water, flow 0.75 mL/min.
- Sample volume 10 µL.
- Standard concentrations: 50, 100, 250 and 500 µg/mL (prepared in phosphate buffered saline, PBS).
- Quality Control Concentration: 150 ng/mL (prepared in PBS).

Under these conditions, the peak of L-ascorbic acid appears at retention time = 2.1 min. The calibration line is linear in the range of concentrations studied.

### Example 1: Preparation of a nonwoven fabric membrane with L-ascorbic acid

A solution of 17 % (by mass) of PLA was prepared in a mixture of 40 % dioxane and 60 % acetone. The solution was stirred during 24h to ensure its regularity. This solution was used for the electrospinning (creation of the fibres) on a cylindrical rotatory collector at a distance of 100 mm and a voltage difference of 15 kV (positive in the needle). The injection flow rate of the polymer solution was 15.5 mL/h. Simultaneously, on the same cylinder and in a disposition at 180° (as shown in FIG.1) the electrospraying of the active agent solution was performed. This solution was prepared by dissolving 2.6 % (by mass) of vitamin C in ethanol and stirring for 6 hours. The electrospray was performed at a distance of 25 mm from the collector and with a voltage of 10 kV (positive in the needle). The 80mm-diameter cylinder, rotated at an angular velocity of 16 min⁻¹. The simultaneous process of electrospinning and electrospraying was maintained during 56 min obtaining membranes with an average thickness of 0.352 mm and with a ratio of active agent in the membrane of 51 % (w/w). FIG. 2 shows images obtained by scanning electron microscopy (SEM) of the membrane obtained at different magnifications.

### Example 2: Preparation of a nonwoven fabric membrane with resveratrol

A solution of 15.8 % (by mass) of PLA was prepared in a mixture of 40 % dioxane and 60 % acetone. The solution was stirred during 24h to ensure its regularity. This solution was used for the electrospinning (creation of the fibres) on a cylindrical rotatory collector at a distance of 100 mm and a voltage difference of 15 kV (positive in the needle). The injection flow rate of the polymer solution was 15.5 mL/h. Simultaneously, on the same cylinder and in a disposition at 180° (as shown in FIG.1) the electrospraying of the active agent solution was performed. This solution was prepared by dissolving 1.47 % (by mass) of resveratrol in ethanol and stirring for 6 hours. The electrospray was performed at a distance of 25 mm from the collector and with a voltage of 10 kV (positive in the needle). The 80mm-diameter cylinder, rotated at an angular velocity of 16 min⁻¹. The simultaneous process of electrospinning and electrospraying was maintained during 60 min obtainig membranes with an average thickness of 0.326 mm and with a ratio of active agent in the membrane of 30 % (w/w). FIG. 3 shows images obtained by scanning electron microscopy (SEM) of the membrane obtained at different magnifications.

### Comparative Example 1: Preparation of a nonwoven membrane with L-ascorbic acid according to the corresponding process to that described in the state of the art (JP2008179629)

A solution of 11.3 % (by mass) of PLA was prepared in a mixture of 40 % dioxane and 60 % acetone. A 5.7 % (by mass) of vitamin C was added to the solution. The solution was stirred during 24h to ensure its regularity. This solution was used for the electrospinning (creation of the fibres with Vitamin C in them) on a cylindrical rotatory collector at a distance of 100mm and a voltage difference of 15 kV (positive in the needle). The injection flow rate of the polymer solution was 15.5 mL/h. The 80mm-diameter cylinder, rotated at an angular velocity of 16 min⁻¹. The process of electrospinning was maintained during 117 min obtaining membranes with an average thickness of 0.306 mm and with a ratio of active agent in the membrane of 33 % (w/w). FIG. 4 shows images obtained by scanning electron microscopy (SEM) of the membrane of the Comparative Example 1 at different magnifications.

### Example 3: Release Assay

In culture plates in plastic material, of 6 wells, 0.50 grams of carbopol gel (carbopol gel 940 to 1 % w/v in distilled water) per well were deposited, and then on the gel a nanofibre membrane (of 15 mm diameter) loaded with 10 mg of L-ascorbic acid (theoretical load) prepared according to Example 1 or a nanofibre membrane (15 mm diameter) loaded with 12 mg of L-ascorbic acid (theoretical load) prepared as Comparative Example 1. In the case of the membrane of the invention, it was tested by HPLC technique that the theoretical charge and the actual total load (calculated as the sum of the amount of L-ascorbic acid released and the amount of L-ascorbic acid remaining in the membrane) were equivalent. The plates were incubated, covered from that moment, at 37 °C. To measure the amount of L-ascorbic acid released at set times (30 minutes in the case of the membrane prepared according to Example 1) and 30, 60, 90 and 120 minutes in the case of the membrane prepared according to Comparative Example 1) the membranes were removed, 9.5 mL of phosphate buffered saline (PBS) were added to each well and they were mixed by pipetting the gel in the same well. From there, a sample of 1 mL was taken and was diluted with 1 mL of PBS in a 12 mL-amber vial. With a 1 mL-disposable syringe, part of the vial content was aspirated and that content was filtered to an HPLC vial with a 0.45µm-disposable filter to remove insolubles compounds. The sample in the vial was injected into the HPLC apparatus under the conditions previously described. The amount of ascorbic acid released at each time was represented as fraction (%) with respect to the total amount (theoretical load of the manufacturing process). Studies were performed in triplicate.

FIG. 4 shows the amount of ascorbic acid released at each time represented as fraction (%) with respect to the theoretical total amount. It has been observed that the release of L-ascorbic acid from the nanofibre membranes of the invention (prepared according to Example 1) was 87 ± 24 % after 30 minutes. Moreover, it was proved by HPLC that the active agent was released in unaltered form. Furthermore, the release of L-ascorbic acid from the nanofibre membranes of the state of the art (prepared according to Comparative Example 1) was inferior to 5 % after 30 minutes, 10.2 ± 0.7 % after 60 minutes, 11.0 ± 0.7 % after 90 minutes, and 12.7 ± 7.0 % after 120 minutes.

### Analysis of results:

While the membranes of the invention are capable of quickly releasing practically the total amount of active agent contained in the membrane when contacted with the gel base, the membranes of the state of art with a comparable loading of active agent are only capable of immediately releasing small fractions (less than 5%) and even with prolonged incubations (120 minutes) only a very moderated release of 13 % is achieved.

### REFERENCES CITED IN THE APPLICATION

JP2008179629

## Claims

1. A nonwoven fabric membrane for topical use on the skin, which comprises polymeric nanofibres and an active agent comprised in an amount from 25 to 80 % by weight with respect to the total weight of the nonwoven fabric membrane, wherein
a) the active agent is soluble in a suitable biocompatible solvent system,
b) the membrane is capable of releasing an amount equal to or greater than 70 % of the total weight of the active agent in a 30-minute period after contacting the membrane with an appropriate amount of the suitable biocompatible solvent system in which the active agent is completely solubilised, and
c) the total content of the active agent is arranged externally to the polymeric nanofibres of the membrane.

2. The nonwoven fabric membrane according to claim 1, further comprising a second active agent, which is soluble in a suitable biocompatible solvent system, wherein the membrane is capable of releasing said second active agent immediately when contacted with an appropriate amount of the suitable biocompatible solvent system in which the second active agent is completely solubilised.

3. The nonwoven fabric membrane according to any of claims 1-2, wherein the polymeric nanofibres are electrospun nanofibres.

4. The nonwoven fabric membrane according to any of claims 1-3, wherein the polymeric nanofibres of the membrane have an average diameter from 50 to 2000 nm.

5. The nonwoven fabric membrane according to any of claims 1-4, wherein the polymeric nanofibres comprise one or more polymers selected among polyglycolic acid, poly-D,L-lactic acid, poly-D,L-lactide-co-glycolide, polycaprolactone, polydioxanone, polyvinyl alcohol, collagen, cellulose, hyaluronic acid, polyamide, polyester, polyurethane, polypropylene, elastanes, and a combination thereof.

6. The nonwoven fabric membrane according to any of claims 1-5, wherein the active agent comprises an amount from 30 to 70 % by weight with respect to the total weight of the nonwoven fabric membrane.

7. The nonwoven fabric membrane according to any of claims 1-6, wherein the active agent is a therapeutic and/or cosmetic agent.

8. The nonwoven fabric membrane according to any of claims 1-7, wherein the active agent is resveratrol or Vitamin C.

9. The nonwoven fabric membrane according to any of claims 1-8, which is capable of releasing an amount which is equal to or greater than 70 % of the total weight of the active agent that is contained in the membrane during a period of 30 minutes when contacting the membrane with an appropriate amount of the suitable biocompatible solvent system in which the active agent is completely solubilised.

10. A process for the preparation of the nonwoven fabric membrane defined in any of claims 1-9, which comprises the following steps:
a) preparing a solution of one or more biocompatible polymers in a suitable solvent system;
b) preparing a solution of the active agent in a suitable solvent system, wherein the polymer or polymers from step a) are insoluble;
c) carrying out the electrospinning of the solution from step a) and, simultaneously, the spray of the solution from step b) with a flow rate of the solution from step b) and during an appropriate time to obtain a nonwoven fabric membrane comprising the active agent in an amount from 25 % to 80 % by weight with respect to the total weight of the nonwoven fabric membrane; and
d) optionally, drying the nonwoven fabric membrane obtained from step c).

11. A kit comprising the nonwoven fabric membrane defined in any of claims 1-9 and a suitable biocompatible solvent system capable of solubilising the active agent of the membrane when contacted therewith.

12. The kit according to claim 11, wherein the biocompatible solvent system comprises one or more solvents selected from the group consisting of water, ethanol, ethylene glycol, propylene glycol, butylene glycol, diethylene glycol, tetraethylene glycol, 1,2,3-propanetriol (glycerin), benzyl alcohol, triglycerids of capric/caprylic acid, vegetable oil, mineral oil, animal fat, fractions and mixtures thereof.

13. The kit according to any of claims 11-12, wherein the biocompatible solvent system is part of a topical composition that further comprises one or more appropriate topical excipients or carriers.

14. The kit according to claim 13, wherein the topical composition is selected from the group consisting of a liquid, a gel, a paste, a cream, an emulsion, a lotion, a foam, a spray, a patch and a stick.

15. A cosmetic or sanitary product comprising the nonwoven fabric membrane defined in any of the claims 1-9.

16. A nonwoven fabric membrane as defined in any of claims 1-9, for use in the treatment of a disease, disorder or abnormal condition of the skin, wherein the active agent is a therapeutic agent.

17. A product as defined in claim 15, for use in the treatment of a disease, disorder or abnormal condition of the skin, wherein the active agent is a therapeutic agent.

18. The nonwoven fabric membrane as defined in any of claims 1-9 or the product defined in claim 15, for use according to any of claims 16-17, wherein the disease, skin disorder or anomalous condition is selected from the group consisting of cellulitis, acne, skin aging, hyperpigmentation, keratosis, dandruff, warts, photodamaged skin, chronic dermatosis, dryness, ichthyosis, wound healing and skin infections caused by viruses, fungi or bacteria.

19. A cosmetic use of the nonwoven fabric membrane according to any of claims 1-9, or of the product according to claim 16, wherein the active agent is a cosmetic agent, as an agent for skin care, where skin care comprises improving at least one of the following symptoms: aging, wrinkles, skin blemishes, cellulite, skin imperfections, roughness, scaling, dehydration, strain, cracking and lack of elasticity.

## Patentansprüche

1. Eine Vliesstoffmembrane zur topischen Anwendung auf die Haut, welche polymere Nanofasern und einen Wirkstoff enthalten in einer Menge von 25 Gew.-% bis 80 Gew.-% bezogen auf das gesamte Gewicht der Vliesstoffmembrane umfasst, wobei
a) der Wirkstoff in einem geeigneten biokompatiblen Lösungsmittelsystem löslich ist,
b) wobei die Membrane eine Menge von 70 % des gesamten Gewichts des Wirkstoffs oder mehr in einem Zeitraum von 30 Minuten nach dem Kontakt der Membran mit einer geeigneten Menge des geeigneten biokompatiblen Lösungsmittelsystems, in dem der Wirkstoff vollständig gelöst wird, freisetzen kann und
c) wobei der gesamte Gehalt des Wirkstoffs außerhalb der polymeren Nanofasern der Membran angeordnet ist.

2. Die Vliesstoffmembrane nach Anspruch 1, weiterhin umfassend einen zweiten Wirkstoff, der in einem geeigneten biokompatiblen Lösungsmittelsystem löslich ist, wobei die Membrane den zweiten Wirkstoff unmittelbar dann freisetzen kann, wenn sie in Kontakt mit einer geeigneten Menge des geeigneten biokompatiblen Lösungsmittelssystems kommt, in dem der zweite Wirkstoff vollständig gelöst wird.

3. Die Vliesstoffmembrane nach einem der Ansprüche 1-2, wobei die polymeren Nanofasern elektrogesponnene Nanofasern sind.

4. Die Vliesstoffmembrane nach einem der Ansprüche 1-3, wobei die polymeren Nanofasern der Membran einen durchschnittlichen Durchmesser von 50 bis 2.000 nm haben.

5. Die Vliesstoffmembrane nach einem der Ansprüche 1-4, wobei die polymeren Nanofasern ein oder mehr Polymere umfassen, die aus Polyglykolsäure, Poly-D,L-Milchsäure, Poly-D,L-Lactid-co-Glycolid, Polycaprolacton, Polydioxanon, Polyvinylalkohol, Kollagen, Cellulose, Hyaluronsäure, Polyamid, Polyester, Polyurethan, Polypropylen, Elastanen, und einer Kombination davon ausgewählt sind.

6. Die Vliesstoffmembrane nach einem der Ansprüche 1-5, wobei der Wirkstoff eine Menge von 30 Gew.-% bis 70 Gew.-% bezogen auf das gesamte Gewicht der Vliesstoffmembrane umfasst.

7. Die Vliesstoffmembrane nach einem der Ansprüche 1-6, wobei der Wirkstoff ein Therapeutikum und/oder ein Kosmetikum ist.

8. Die Vliesstoffmembrane nach einem der Ansprüche 1-7, wobei der Wirkstoff Resveratrol oder Vitamin C ist.

9. Die Vliesstoffmembrane nach einem der Ansprüche 1-8, welche eine Menge von 70 % oder mehr des gesamten Gewichts des Wirkstoffs, der in der Membran enthalten ist, in einem Zeitraum von 30 Minuten freisetzen kann, wenn die Membran in Kontakt mit einer geeigneten Menge des geeigneten biokompatiblen Lösungsmittels freisetzen kann, in dem der Wirkstoff vollständig gelöst wird.

10. Ein Verfahren zur Herstellung der in einem der Ansprüche 1-9 definierten Vliesstoffmembrane, welches folgende Schritte umfasst:
a) herstellen von einer Lösung von einem oder mehreren biokompatiblen Polymeren in einem geeigneten Lösungsmittelsystem;
b) herstellen von einer Lösung des Wirkstoffs in einem geeigneten Lösungsmittelsystem, wobei das Polymer oder die Polymere des Schritts a) unlöslich sind;
c) durchführen des Elektrospinnens der Lösung von Schritt a) und, gleichzeitig, das Sprühen der Lösung von Schritt b) mit einer Flussrate der Lösung von Schritt b) und während eines geeigneten Zeitraums, um eine Vliesstoffmembrane umfassend den Wirkstoff in einer Menge von 25 Gew.-% bis 80 Gew.-% bezogen auf das gesamte Gewicht der Vliesstoffmembrane zu erhalten; und
d) wahlweise, trocknen der aus dem Schritt c) erhaltenen Vliesstoffmembrane.

11. Ein Kit umfassend die in einem der Ansprüche 1-9 definierte Vliesstoffmembrane und ein geeignetes biokompatibles Lösungsmittelsystem, das den Wirkstoff der Membran lösen kann, wenn es in Kontakt damit kommt.

12. Der Kit nach Anspruch 11, wobei das biokompatible Lösungsmittelsystem ein oder mehrere Lösungsmittel umfasst, die aus der Gruppe bestehend aus Wasser, Ethanol, Ethylenglykol, Propylenglylol, Butylenglykol, Diethylenglykol, Tetraethylenglykol, 1,2,3-Propanetriol (Glycerin), Benzylalkohol, Triglyceriden von Caprin-/Caprylsäure, Pflanzenöl, Mineralöl, Tierfett, Fraktionen und Mischungen davon ausgewählt sind.

13. Der Kit nach einem der Ansprüche 11-12, wobei das biokompatible Lösungsmittelsystem Teil von einer topischen Zusammensetzung ist, die einen oder mehrere geeignete topische Hilfsstoffe oder Trägerstoffe umfasst.

14. Der Kit nach Anspruch 13, wobei die topische Zusammensetzung ausgewählt aus der Gruppe bestehend aus einer Flüssigkeit, einem Gel, einer Paste, einer Lotion, einem Schaum, einem Spray, einem Pflaster und einem Stift ist.

15. Ein kosmetisches Produkt oder ein Hygieneprodukt umfassend die in einem der Ansprüche 1-9 definierte Vliesstoffmembrane.

16. Eine Vliesstoffmembrane wie in einem der Ansprüche 1-9 definiert, zur Verwendung in der Behandlung von einer Krankheit, einer Störung oder einem abnormalen Zustand der Haut, wobei der Wirkstoff ein Therapeutikum ist.

17. Ein Produkt wie in Anspruch 15 definiert, zur Verwendung in der Behandlung von einer Krankheit, einer Störung oder einem abnormalen Zustand der Haut, wobei der Wirkstoff ein Therapeutikum ist.

18. Die Vliesstoffmembrane wie in einem der Ansprüche 1-9 definiert oder das Produkt definiert in Anspruch 15, zur Verwendung nach einem der Ansprüche 16-17, wobei die Krankheit, die Hautstörung oder der abnormale Zustand ausgewählt ist aus der Gruppe bestehend aus Zellulitis, Akne, Hautalterung, Hyperpigmentierung, Keratose, Schuppen, Warzen, lichtgeschädigter Haut, chronischer Dermatose, Trockenheit, Ichthyose, Wundheilung und durch Viren, Pilze oder Bakterien verursachten Hautentzündungen.

19. Eine kosmetische Verwendung der Vliesstoffmembrane nach einem der Ansprüche 1-9, oder des Produkts nach Anspruch 16, wobei der Wirkstoff ein kosmetischer Wirkstoff ist, als Stoff zur Hautpflege, wobei die Hautpflege die Besserung von mindestens einer der folgenden Symptome umfasst: Alterung, Falten, Hautunreinheiten, Zellulitis, Hautunregelmäßigkeiten, Hautrauheit, schuppender Haut, Hautaustrocknung, Belastung, Rissbildung und mangelnder Elastizität.

## Revendications

1. Une membrane de textile non tissé pour l'utilisation topique sur la peau, qui comprend des nanofibres polymères et un agent actif compris dans une quantité de 25 à 80 % en poids par rapport au poids total de la membrane de tissu non tissé, dans laquelle
a) l'agent actif est soluble dans un système solvant biocompatible approprié,
b) la membrane peut libérer une quantité égale ou supérieure à 70 % du poids total de l'agent actif dans une période de 30 minutes après le contact de la membrane avec une quantité appropriée du système solvant biocompatible approprié dans lequel l'agent actif est complètement solubilisé, et
c) le contenu total de l'agent actif est disposé à l'extérieur des nanofibres polymères de la membrane.

2. La membrane de textile non tissé selon la revendication 1, comprenant en outre une deuxième agent actif qui est soluble dans un système solvant biocompatible approprié, où la membrane peut libérer ledit deuxième agent actif immédiatement lors du contact avec une quantité appropriée du système solvant biocompatible approprié dans lequel le deuxième agent actif est complètement solubilisé.

3. La membrane de textile non tissé selon l'une quelconque des revendications 1-2, dans laquelle les nanofibres polymères sont des nanofibres électrofilées.

4. La membrane de textile non tissé selon l'une quelconque des revendications 1-3, dans laquelle les nanofibres polymères de la membrane ont un diamètre moyen de 50 à 2.000 nm.

5. La membrane de textile non tissé selon l'une quelconque des revendications 1-4, dans laquelle les nanofibres polymères comprennent un ou plusieurs polymères choisis parmi l'acide polyglicolique, acide poly-D,L-lactique, poly-D,L-lactide-co-glycolide, polycaprolactone, polydioxanone, alcool polyvinylique, collagène, cellulose, acide hyaluronique, polyamide, polyester, polyuréthane, polypropylène, elastanes, et une combinaison de ceux-ci.

6. La membrane de textile non tissé selon l'une quelconque des revendications 1-5, dans laquelle l'agent actif comprend/est compris dans***NOTA*** une quantité de 30 à 70 % en poids par rapport au poids total de la membrane de tissu non tissé.

7. La membrane de textile non tissé selon l'une quelconque des revendications 1-6, dans laquelle l'agent actif est un agent thérapeutique et/ou cosmétique.

8. La membrane de textile non tissé selon l'une quelconque des revendications 1-7, dans laquelle l'agent actif est resvératrol ou vitamine C.

9. La membrane de textile non tissé selon l'une quelconque des revendications 1-8, qui peut libérer une quantité qui est égale ou supérieure à 70 % du poids total de l'agent actif qui est contenu dans la membrane pendant une période de 30 minutes lors du contact de la membrane avec une quantité appropriée du système solvant biocompatible approprié dans lequel l'agent actif est complètement solubilisé.

10. Un procédé de préparation de la membrane de textile non tissé définie dans l'une quelconque des revendications 1-9, qui comprend les étapes suivantes :
a) préparer une solution d'un ou de plusieurs polymères biocompatibles dans un système solvant approprié ;
b) préparer une solution de l'agent actif dans un système solvant approprié, où le polymère ou polymères de l'étape a) sont insolubles ;
c) effectuer l'électrofilage de la solution de l'étape a) et, simultanément, la pulvérisation de la solution de l'étape b) avec un débit de la solution de l'étape b) et pendant une période appropriée pour obtenir une membrane de textile non tissé comprenant l'agent actif dans une quantité de 25 % à 80 % en poids par rapport au poids total de la membrane de textile non tissé ; et
d) facultativement, sécher la membrane de textile non tissé obtenue à partir de l'étape c).

11. Un kit comprenant la membrane de textile non tissé définie dans l'une quelconque des revendications 1-9 et un système solvant biocompatible approprié qui peut solubiliser l'agent actif de la membrane lors du contact avec celle-ci.

12. Le kit selon la revendication 11, dans lequel le système solvant biocompatible comprend un ou plusieurs solvants choisis dans le groupe constitué d'eau, éthanol, éthylène glycol, propylène glycol, butylène glycol, diéthylène glycol, tétraéthylène glycol, 1,2,3-propanetriol (glycérol), alcool benzylique, triglycérides d'acide caprique/caprylique, huile végétal, huile minéral, graisse animale, fractions et mélanges de ceux-ci.

13. Le kit selon l'une quelconque des revendications 11-12, dans lequel le système solvant biocompatible fait partie d'une composition topique qui comprend en outre un ou plusieurs excipients ou véhicules topiques appropriés.

14. Le kit selon la revendication 13, dans lequel la composition topique est choisie dans le groupe constitué d'un liquide, un gel, une pâte, une crème, une émulsion, une lotion, une mousse, un spray, un timbre et un bâton.

15. Un produit cosmétique ou sanitaire comprenant la membrane de textile non tissé définie dans l'une quelconque des revendications 1-9.

16. Une membrane de textile non tissé telle que définie dans l'une quelconque des revendications 1-9, pour l'utilisation dans le traitement d'une maladie, un trouble ou une condition anormale de la peau, dans laquelle l'agent actif est un agent thérapeutique.

17. Un produit tel que défini dans la revendication 15, pour l'utilisation dans le traitement d'une maladie, un trouble ou une condition anormale de la peau, dans lequel l'agent actif est un agent thérapeutique.

18. La membrane de textile non tissé telle que définie dans l'une quelconque des revendications 1-9 ou le produit défini dans la revendication 15, pour l'utilisation selon l'une quelconque des revendications 16-17, où la maladie, le trouble ou la condition anormale de la peau est choisi(e) dans le groupe constitué de la cellulite, l'acné, le vieillissement cutané, l'hyperpigmentation, la kératose, les pellicules, les verrues, la peau photoendommagée, la dermatose chronique, la sécheresse, l'ichtyose, la guérison de blessures et des infections cutanées causées par des virus, des fonges ou des bactéries.

19. Une utilisation cosmétique de la membrane de textile non tissé selon l'une quelconque des revendications 1-9, ou du produit selon la revendication 16, dans laquelle l'agent actif est un agent cosmétique, comme agent pour le soin de la peau, dans laquelle le soin de la peau comprend améliorer au moins une des symptômes suivants : le vieillissement, les rides, les défauts de la peau, la cellulite, les imperfections de la peau, la rugosité, la desquamation, la déshydratation, le stress**, la gerçure et la manque d'élasticité.
